# EUROPEAN PATENT SPECIFICATION

(11) **EP 1 658 951 B1**
(45) Date of publication and mention of the grant of the patent: **21.09.2011**
(21) Application number: 04745254.5
(22) Date of filing: 18.05.2004
(51) Int. Cl.: B29C 47/92, B29C 47/06, B29C 47/28

(54) **EXTRUSION MOLDING APPARATUS FOR RESIN TUBE**
EXTRUSIONSFORMVORRICHTUNG FÜR HARZROHR
APPAREIL DE MOULAGE PAR EXTRUSION POUR TUBE EN RESINE

(30) Priority: 27.08.2003 JP 2003303095
(43) Date of publication of application: 24.05.2006
(73) Proprietor: Pla Giken Co., Ltd., Suita-shi, Osaka 564-0051 (JP)
(72) Inventor: KIKUSAWA, Yoshiharu, Suita-shi, Osaka 564-0051 (JP)
(74) Representative: Hering, Hartmut
(86) International application number: PCT/JP2004/006655
(87) International publication number: WO 2005/023516

(56) References cited:
- EP-A- 0 987 044
- WO-A-01/89802
- WO-A-98/56447
- WO-A-03/023791
- JP-A- 4 212 377
- JP-A- 5 162 186
- JP-A- 63 007 926
- JP-A- 2001 088 199
- JP-A- 2001 269 411
- JP-A- 2001 277 330
- JP-A- 2002 277 330

## Description

### TECHNICAL FIELD

The present invention relates to an extrusion molding apparatus for a resin tube, designed to mold a tube used as a material for catheters or the like, by causing thermally melted resin being extruded from an extruder to pass through a die.

### BACKGROUND ART

As for said extrusion molding apparatus for a resin tube, there have heretofore been ones shown in the following Patent Documents 1 and 2. According to these gazettes, said extrusion molding apparatus comprises extruders for thermally melting resins to enable the extrusion of the resins, and a die having tube molding passages causing the resins extruded from said extruders to forwardly pass therethrough to enable the molding of a tube.

And, during the operation of said extrusion molding apparatus, said extruders are driven so that thermally melted resins are extruded from the extruders. Thereupon, the resins pass through said tube molding passages, whereby a tube is molded.

Further, said extrusion molding apparatus enables automatic operation, wherein in order that the flow per unit time of the resin passing from the extruder to the tube molding passage may be incessantly changed, said flow is made adjustable. During this automatic operation, the flow of the resin being extruded from said extruder is changed. Thereupon, following this change, the flow of the resin passing through said tube molding passage is changed so as to enable the molding of a tube to desired dimensions, concerning wall thickness and diameter.

More specifically, for example, if the flow of the resin being extruded from said extruder is increased, the wall thickness of the tube molded at this time increases or the diameter increases. On the other hand, if the flow from said extruder is reduced, the wall thickness of the tube molded at this time becomes thinner or the diameter is reduced. And, in this manner, the molding of a tube is enabled such that any cross-section along the longitudinal direction has a desired dimension.

- Patent Document 1:: Japanese Patent Laid-Open Gazsette No. Hei 4-212377.
- Patent Document 2:: Japanese Patent Laid-Open Gazette No. 2001-88199.
EP 0 987 044 A2 relates to tubing and a method for making the same, wherein a valve member is provided for directing the resin flowing into a chamber to the outlet. A modulator is provided for regulating the flow rate of resins.
WO 03/023791 A relates to an extrusion apparatus for producing a cable, the core of which is coated with a pair of coating layers made of different kinds of material. Dies are coaxially mounted between each other around the same longitudinal axis which is substantially parallel to the advancing direction of the core and an extrusion head comprises conveyor elements associated with the respective dies.
WO 98/56447 A discloses an apparatus for manufacturing a guiding catheter having controlled flexibility, wherein an extrusion head comprises first and second extrusion ports providing entries for second and first materials, respectively.
JP 2001 269411 A discloses a tube for a catheter which is formed by causing two resins having different hardness to continue in a longitudinal direction in which the reliability of junction is raised by relaxing the changes of hardness on the boundary surface of the resin.
The closest prior art document WO 01/89802 A and the corresponding US-Patent US 6,808,308 B1 disclose an apparatus for manufacturing stiffness-taper tubing having the features described in the preamble of claim 1.

### DISCLOLSURE OF THE INVENTION

### PROBLEMS TO BE SOLVED BY THE INVENTION

In this connection, the passage for resin flow from said extruder to the tube molding passage in the die has a large volume to a certain extent as a whole. And, during the operation of said extrusion molding apparatus, resin is filled in said passage.

During the automatic operation of said extrusion molding apparatus, if the flow of the resin being extruded from the extruder is increased, the resin in said passage is pressurized on the basis thereof, this pressure being transmitted as far as to the resin in said tube molding passage. Thereby, the flow of the resin going to pass through this tube molding passage tends to be increased.

Here, as described above, the passage has a large volume and is filled with resin, and the volume of the resin also increases. For this reason, this resin tends to be subjected to volumetric variation to greatly contract by the pressure from said extruder. Consequently, there is a possibility that the transmission of said pressure from said extruder to the tube molding passage may delay by an amount corresponding thereto. Particularly, when said resin is soft, said volumetric variation due to said pressure increases, leading to a possibility that the transmission of this pressure may further delay.

Further, as described above, the volume of the resin filled in the passage is large. For this reason, when the flow is changed in such a manner as to reduce the flow of the resin being extruded from the extruder, the resin in said passage tends to be subjected to volumetric variation such that it is expanded by the residual pressure. Consequently, even if the flow of the resin being extruded from the extruder is reduced, the flow of the resin passing through said tube molding passage does not become instantly reduced, that is, there is a possibility that problems may develop concerning responsiveness. Particularly, when said resin is soft, the volumetric variation further increases, making said problems concerning responsiveness more remarkable.

As a result, with the conventional extrusion molding apparatus, it is difficult to cause the change of the flow of the resin passing through said tube molding passage to follow with satisfactory responsiveness the change of the flow of the resin being extruded from the extruder. Thus, it is not easy to make more accurate the dimensional accuracy of the tube being molded.

### MEANS FOR SOLVING PROBLEMS

With the above in mind, an object of the invention is to make more accurate the dimensions of a tube being molded by an extrusion molding apparatus.

Further, another object of the invention is to simplify the arrangement of the extrusion molding apparatus enabling the molding of the accurate tube described above.

Further, it is also an object to ensure that the molding of the accurate tube described above is easily performed.

The invention provides an extrusion molding apparatus for a resin tube, comprising a plurality of extruders for thermally melting and extruding resins of different kinds, and a die provided with an inner layer tube molding passage for forwardly passing therethrough the resin extruded from one extruder of these extruders to enable the molding of an inner layer tube, and an outer layer tube molding passage for forwardly passing therethrough the resin extruded from the other extruder to enable the molding of an outer layer tube which is to be externally fitted integrally on said inner layer tube, said die enabling the molding of a multi-layer tube by these inner and outer layer tubes, said die being formed with inflow passages enabling the resins extruded from said extruders to flow into the respective rears of said tube molding passages, wherein flow adjusting valves are installed which enable the adjustment of the respective flows per unit time of the resins extruded from said extruders and passed said inner and outer layer tube molding passages, said flow adjusting valves make openable/closable the communication passage for communicating the intermediate portion of said inflow passages to outside said die, according to the preamble of claim 1.

Opening-degree adjusting valves are installed which enable the adjustment of the respective degrees of opening of said communication passages, according to the characterising part of claim 1.

Further, in said invention, inner and outer extrusion ports constituting the respective front ends of said inner and outer layer tube molding passages may be disposed radially close to each other and be opened forwardly from the front end of the die separately from each other.

Further, added to said invention, there is provided an extrusion molding apparatus for a resin tube, with said die formed with a through-hole longitudinally extending through said die and passing inwardly of said inner layer tube molding passage, said tube being externally fitted on a core material forwardly passing through said through-hole, wherein
said inner extrusion port of said inner layer tube molding passage may be disposed close to the front end opening radially constituting the front end of said through-hole.

### EFFECTS OF THE INVENTION

The effects by the invention are as follows.

The invention provides an extrusion molding apparatus for a resin tube comprising a plurality of extruders for thermally melting and respectively extruding resins of different kinds, and a die provided with an inner layer tube molding passage for forwardly passing therethrough the resin extruded from one of these extruders to enable the molding of an inner layer tube, and an outer layer tube molding passage for forwardly passing therethrough the resin extruded from the other extruder to enable the molding of an outer layer tube which is to be externally fitted integrally on said inner layer tube, said die enabling the molding of a multi-layer tube by these inner and outer layer tubes, wherein there are installed flow adjusting valves respectively enabling the adjustment of each flow per unit time of each resin extruded from each said extruder and passed to one of said inner and outer layer tube molding passages.

With the arrangement thus made, in the case of molding a multi-layer tube by the driving of each said extruder to cause each resin extruded from each extruder to pass through each said tube molding passage, the flow of said resin can be adjusted by the actuation of each said flow adjusting valve. Consequently, the wall thickness and diameter of said inner and outer layer tubes can be adjusted to respective desired values, providing a desired multi-layer tube.

Here, the volume of the space in each "passage" for resin flow extending from each said flow adjusting valve to each tube molding passage is smaller than that of such "passage" extending from each extruder to each tube molding passage. For this reason, the volume of the resin filled in each said "passage" becomes also small. Consequently, by the amount corresponding thereto the volumetric variation of said resin due to external force is suppressed such that it is small.

And, suppose that each said flow adjusting valve is actuated so as to change the flow of the resin passing from each said flow adjusting valve to each said tube molding passage. In this case, as described above, the volume of the resin in each "passage" is small and volumetric variations due to external force are suppressed such that they are small. For this reason, the change of the flow of the resin passing through each said tube molding passage follows the actuation of each said flow adjusting valve with satisfactory responsiveness. Consequently, the dimensional accuracies of the inner and outer layer tubes of the multi-layer tube being molded by the extrusion molding apparatus can be respectively made further high.
Further, each said flow adjusting valve makes openable/closable the communication passage communicating the intermediate portion of each said inflow passage to outside the die.
For this reason, when said resin is passed from said extruder through the flow adjusting valve and inflow passage to said tube molding passage, a partial flow of the full flow extruded from said extruder is discharged in a predetermined amount to outside the die through said communication passage by said flow adjusting valve. Thereupon, thereby, the adjustment of the flows of the resins passed to said tube molding passages is made possible.
That is, even in the case where the adjustment of the flow of the resin passed to said tube molding passage is made possible, the full flow extruded from said extruder can be made substantially constant. For this reason, in the case where said tube is to be molded to desired dimensions, trying to change the flow extruded from the extruder would tend to make the control troublesome; however, such control is unnecessary. Consequently, the molding of said accurate tube is facilitated.
Further, there are provided opening-degree adjusting valves making adjustable the degree of opening of each said communication passage.
For this reason, partial flow of the resin passing through said communication passage to be discharged to outside the die can be made to have a desired value by the adjustment of the degree of opening of the communication passage by said opening-degree adjusting valve. And, since such adjusting operation can be facilitated, the molding of a tube of desired dimensions is further facilitated.

Further, in said invention, inner and outer extrusion ports constituting the respective front ends of said inner and outer layer tube molding passages may be disposed radially close to each other and be opened at the front end surface of the die separately from each other.

With the arrangement thus made, each resin being extruded from each said extruder by the driving of each said extruder is passed through each tube molding passage in said die, thereby molding inner and outer layer tubes. Further, when the inner and outer layer tubes are extruded forwardly of the die through said inner and outer extrusion ports, the outer layer tube is externally fitted on the inner layer tube, thereby molding an integral multi-layer tube.

In the above case, the inner and outer extrusion ports are disposed radially close to each other. For this reason, when said individual resins pass through the tube molding passages in said die and are forwardly extruded from said inner and outer extrusion ports, said inner and outer layer tubes immediately after being forwardly extruded from said inner and outer extrusion ports fit together and are smoothly integrated without requiring relatively large radial deformation.

Furthermore, as described above, the inner and outer extrusion ports are partly or wholly opened forwardly from the front end surface of said die separately from each other. For this reason, when said inner and outer layer tubes fit together, said inner and outer layer tubes are suppressed from pressing each other.

Consequently, said inner and outer layer tubes are prevented from being unintentionally deformed by mutual pressing. For this reason, the respective wall thicknesses of the inner and outer layers of the multi-layer tube molded by said extrusion molding apparatus can be respectively made more accurate.

Further, added to said invention, there is provided an extrusion molding apparatus for a resin tube with said die formed with a through-hole longitudinally extending through said die and passing inwardly of said inner layer tube molding passage, said tube being externally fitted on a core material forwardly passing through said through-hole, wherein
said inner extrusion port of said inner layer tube molding passage may be disposed close to the front end opening radially constituting the front end of said through-hole.

With the arrangement thus made, by the driving of each said extruder, a multi-layer tube is molded as it is extruded from said die, and this tube is externally fitted on said core material, so that an intermediate product is molded using these tubes and the core material.

Here, as described above, the inner extrusion port is disposed radially close to said front end opening. Furthermore, as described above, the inner and outer extrusion ports are disposed radially close to each other. For this reason, when said tube is extruded from said inner extrusion port forwardly of said die, said inner and outer layer tubes immediately after they are extruded from said inner extrusion port are, without requiring large radial deformation, externally fitted on the core material immediately after slipping out of the front end opening in said through-hole.

Consequently, the multi-layer tube in said intermediate product molded by said extrusion molding apparatus can have the wall thicknesses of its inner and outer layer tubes respectively made accurate.

### BRIEF DESCRIPTION OF THE DRAWINGS

- [Fig. 1]: a side sectional view of an extrusion molding apparatus.
- [Fig. 2] a: partial enlarged sectional view of Fig. 1.
- [Fig. 3]: a sectional view taken along the line 3-3 in Fig. 1.
- [Fig. 4]: a sectional view of an intermediate product.
- [Fig. 5]: a sectional view of another intermediate product.

### DESCRIPTION OF THE REFERENCE CHARACTERS

- 1: Extrusion molding apparatus
- 2: Tube
- 2a: Inner layer tube
- 2b: Outer layer tube
- 3: Resin
- 4: Resin
- 6: Extruder
- 7: Extruder
- 9: Tube molding passage
- 10: Tube molding passage
- 11: Die
- 16: Axis
- 17: Extrusion port
- 18: Extrusion port
- 19: Front end surface
- 21: Inflow passage
- 22: Inflow passage
- 24: Through-hole
- 25: Core material
- 26: Front end opening
- 29: Die hole
- 30: Another die
- 34: Flow adjusting valve
- 35: Flow adjusting valve
- 36: Valve main body
- 37: Valve body fit hole
- 38: Axis
- 39: Valve body
- 41: First valve hole
- 42: Second valve hole
- 43: Communication passage
- 44: Opening degree adjusting valve
- 47: Intermediate molded article
- R: Turning
- QT: Full flow
- Q1: Partial flow
- Q2: Remainder flow

### BEST MODE FOR CARRYING OUT THE INVENTION

A best mode for carrying out the invention to realize an object which, relating to an extrusion molding apparatus for a resin tube according to the invention, is to make more accurate the dimensions of a tube molded by the extrusion molding apparatus, is as described in claim 1.

### EMBODIMENTS

To describe the invention in more detail, embodiments thereof will be described with reference to the accompanying drawings.

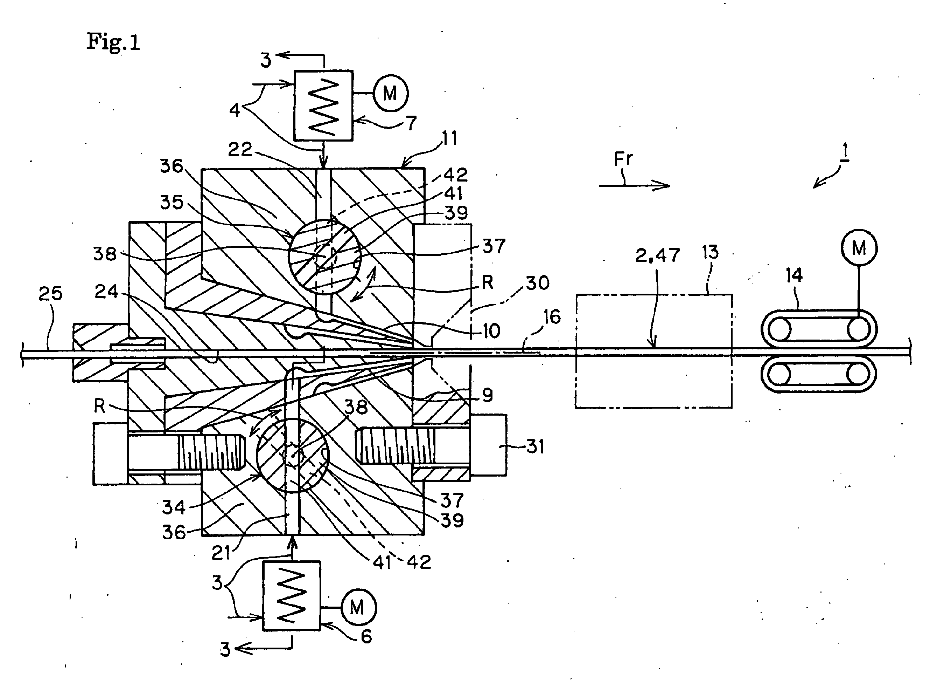

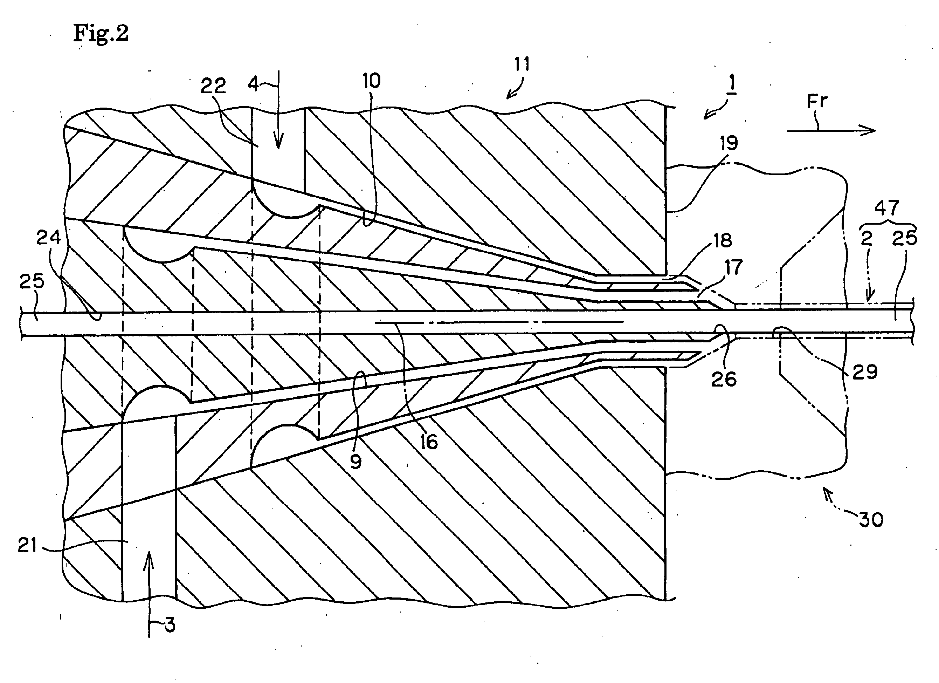

In Figs. 1 - 3, the character 1 denotes an extrusion molding apparatus. This extrusion molding apparatus 1 is used for extrusion-molding a resin multi-layer tube 2 of circular section made of resin. This tube 2 comprises an inner layer tube 2a constituting the inner layer thereof, and an outer layer tube 2b constituting the outer layer of said tube 2 and externally fitted on said inner layer tube 2a to be integrally fixed to the outer peripheral surface of this inner layer tube 2a. Said tube 2 is used, e.g., as a material for catheters and its outer diameter is 1.0 - 1.5 mm. Further, the arrow Fr in the figure indicates the forward direction of extrusion of the tube 2 by said extrusion molding apparatus 1.

The extrusion molding apparatus 1 comprises a plurality (two) of first and second extruders 6 and 7 for thermally melting thermoplastic first and second resins 3 and 4 to enable their extrusion, a die 11 having inner and outer layer tube molding passages 9 and 10 through which the first and second resins 3 and 4 extruded from these first and second extruders 6 and 7 are separately forwardly passed to enable the molding of the inner and outer layer tubes 2a and 2b of said tube 2, a cold-curing device 13 for cold-curing by water said tube 2 molded by being passed through said inner and outer layer tube molding passages 9 and 10, and an electrically driven take-up device 14 for taking up said tube 2 cured by this cold-curing device 13, at a predetermined speed (for example, 2.5 - 10 m/min).

Said first and second resins 3 and 4 differ from each other in hardness at ordinary temperature. Further, the thermally melting of said first and second resins 3 and 4 is achieved by heating using a heater. Further, said first and second extruders 6 and 7 rotationally drive screws by an electric motor.

Said die will now be described in more detail. Said inner and outer tube molding passages 9 and 10 are each in the form of a forwardly tapering frustum and are disposed on the same axis 16. Further, in the radial direction (orthogonal direction, hereinafter the same) of this axis 16, the inner layer tube molding passage 9 is disposed inwardly of the outer layer tube molding passage 10. The respective front ends of said inner and outer layer tube molding passages 9 and 10 are constituted by inner and outer extrusion ports 17 and 18, these extrusion ports 17 and 18 extending substantially in parallel with axis 16. These inner and outer extrusion ports 17 and 18 enable said first and second resins 3 and 4 to be extruded forwardly which is outside said die 11. Said inner and outer extrusion ports 17 and 18 are disposed radially close to each other so as to be close to each other. Further, said inner and outer extrusion ports 17 and 18 are partly or wholly opened forwardly from the front end surface 19 of said die 11 and separately from each other.

Said die 11 is formed with first and second inflow passages 21 and 22. These inflow passages 21 and 22 enables the first and second resins 3 and 4 extruded from said first and second extruders 6 and 7 to flow into the respective rears of said inner and outer layer tube molding passages 9 and 10 separately from each other. In this case, the cross-sectional areas of the two inflow passages 21 and 22 are substantially the same. In addition, the cross-sectional area of the first inflow passage 21 may be made larger than that of the second inflow passage, and vise versa.

The first resin 3 extruded from the first extruder 6, which is one extruder 6 of said first and second extruders 6 and 7, is caused to flow into the rear of said inner layer tube molding passage 9 via said first inflow passage 21. And, thereafter, said resin 3 is passed through said inner layer tube molding passage 9 and extruded forwardly of said die 11, whereby said inner layer tube 2a is molded. Further, the second resin 4 extruded from the second extruder 7 which is the other extruder 7 is caused to flow into the rear of said outer layer tube molding passage 10 via said second inflow passage 22. And, thereafter, said resin 4 is passed through said outer layer tube molding passage 10 and extruded forwardly of said die 11, whereby said outer layer tube 2b is molded. In this case, this outer layer tube 2b is externally fitted integrally on said inner layer tube 2a. In other words, said first and second resins 3 and 4 are passed through the inner and outer layer tube molding passages 9 and 10 via said first and second inflow passages 21 and 22, whereby said tube 2 is molded.

A through-hole 24 of circular cross-section extending on said axis 16 is formed in said die 11. Said through-hole 24 longitudinally extends through said die 11 and is formed inwardly of said inner layer tube molding passage 9. A core material 25 of circular cross-section made of copper metal is allowed to forwardly pass through said through-hole 24. The inner diameter of said through-hole 24 is substantially equal to the outer diameter of said core material 25. And, the inner layer tube 2a of said tube 2 is externally fitted on the core material 25 passing forwardly in said through-hole 24, and said inner layer tube 2a can closely adhere to said core material 25. Disposed radially of said axis 16 and in the vicinity of the front end opening 26 constituting the front end of said through-hole 24 is the inner extrusion port 17 of said inner layer tube molding passage 9.

Installed on said axis 16 is another die 30 having a die hole 29 communicating with the extrusion ports 17 and 18 of said tube molding passages 9 and 10. This another die 30 is removably fixed to the front end surface 19 of said die 11 by a fastener 31.

First and second flow adjusting valves 34 and 35 are installed. These first and second flow adjusting valves 34 and 35 make it possible to separately adjust the respective flows per unit time (m³/min: hereinafter referred to simply as flows) of the first and second resins 3 and 4 extruded from said first and second extruders 6 and 7 and passed to said inner and outer layer tube molding passages 9 and 10.

Further, said flow adjusting valves 34 and 35 adjust the degrees of opening of said inflow passages 21 and 22 to make it possible to adjust the flows of the resins 3 and 4. Concretely, said flow adjusting valves 34 and 35 each comprise a valve main body 36 constituted by part of said die 11, a columnar valve body 39 fitted in a circular valve body fit hole 37 to divide the longitudinal intermediate portion of each of the inflow passages 21 and 22, in such a manner as to allow its turn R around the axis 38, and an actuator 40, such as an air cylinder, which enables this valve body 39 to make turn R to a predetermined turn position. The valve bodies 39 are formed with first and second valve holes 41 and 42 radially extending therethrough and independent of each other. Formed in each valve body 39 is a communication passage 43 communicating the intermediate portion of said second valve hole 42 to outside the die 11. Further, there is installed a needle valve type opening degree adjusting valve 44 which makes it possible to manually adjust the degree of opening of said communication passage 43.

The electric motors for said extruders 6 and 7 and take-up device 14, and actuators 40 are connected to an electronic controller and automatically controlled according to a predetermined program. Here, said extruders 6 and 7 are driven such that when the pressures of the resins 3 and 4 immediately after their extrusion have predetermined values, the flows of the resins 3 and 4 extruded from the extruders 6 and 7 are substantially constant.

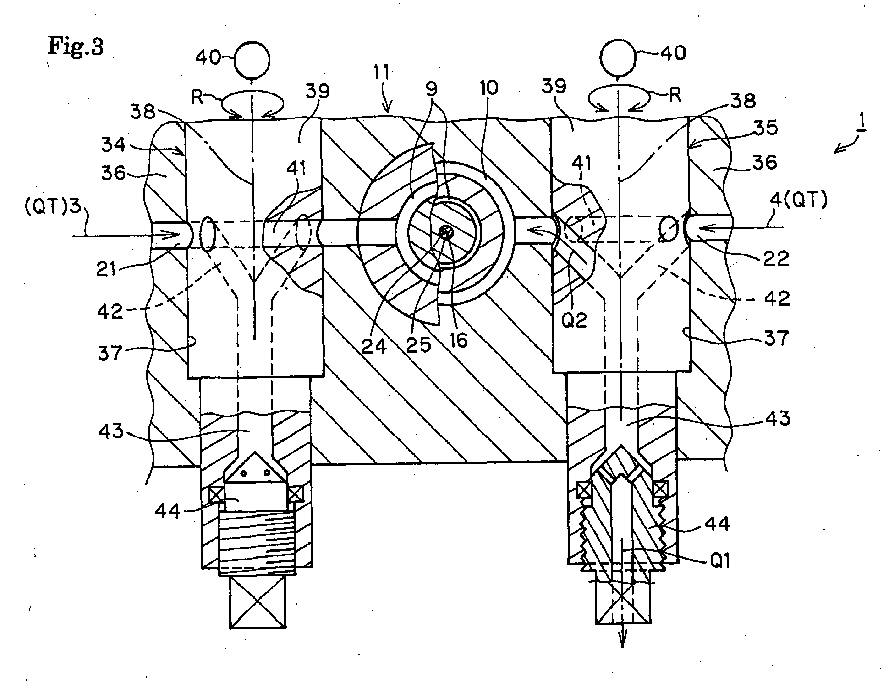

When the flow adjusting valves 34 and 35 are actuated by the driving of said actuators 40, said valve body 39 is turned as at R. And, when this valve body 39 is positioned at "full open position" (in Figs. 1 and 3, the state of the valve body 39 of the first flow adjusting valve 34), said first valve holes 41 provide communication between the divided ends of the inflow passages 21 and 22. Thereupon, the respective full flows (QT) of the resins 3 and 4 extruded from the extruders 6 and 7 are passed through said inflow passages 21 and 22 and the first valve holes 41 to said tube molding passages 9 and 10.

On the other hand, when the driving of said actuator 40 positions said valve body 39 at "half-open position" (in Figs. 1 and 3, the state of the valve body 39 of the second flow adjusting valve 35), said second valve holes 42 provide communication between the divided ends of the inflow passages 21 and 22. And, partial flows (Q1) of the full flows (QT) of the resins 3 and 4 extruded from said extruders 6 and 7 pass through said communication passages 43 and opening-degree adjusting valves 44 and are discharged to outside the die 11, while remainder flows (Q2 = QT - Q1) pass through said inflow passages 21 and 22 and second valve holes 42 to the tube molding passages 9 and 10. In this case, the degree of opening of said communication passage 43 can be adjusted by the operation of said opening-degree adjusting valve 44. This adjustment enables the adjustment of the remainder flows (Q2) passed to the tube molding passages 9 and 10.

Further, though not illustrated, when said valve bodies 39 are positioned at "full close position" by the driving of said actuators 40, the first and second valve holes 41 and 42 are closed by the inner peripheral surfaces of the valve body fit holes 37. In other words, said inflow passages 21 and 22 are fully closed. Thereupon, the flows of the resins 3 and 4 extruded from the extruders 6 and 7 and passed to the tube molding passages 9 and 10 are reduced to zero. In other words, in this manner, the degrees of opening of the inflow passages 21 and 22 are made adjustable.

Further, as described above, when the valve bodies 39 are positioned at said "half open position" by the driving of the actuators 40, the communication passages 43 which communicate the intermediate portions of the inflow passages 21 and 22 to outside the die 11 are opened. When the valve bodies 39 are shifted from this state to said "full open position" or "full close position," the communication passages 43 are closed.

In the case of molding the tube 2 by operating the extrusion molding apparatus 1, first, the extruders 6 and 7 and the take-up device 14 are driven. Further, in this case, the actuators 40 for the flow adjusting valves 34 and 35 are made able to be driven. The resins 3 and 4 extruded from the extruders 6 and 7 attending said driving pass through the inflow passages 21 and 22 and flow adjusting valves 34 and 35 to the inner and outer layer tube molding passages 9 and 10. And, the resins 3 and 4 are passed through the inner and outer layer tube molding passages 9 and 10 and extruded forwardly of the die 11, whereby the inner and outer layer tubes 2a and 2b are molded. Further, when these inner and outer layer tubes 2a and 2b are extruded from the extrusion ports 17 and 18, the outer layer tube 2b is externally fitted on the inner layer tube 2a and they are integrally fixed to each other, so that the multi-layer tube 2 is molded.

Further, simultaneously with the molding of the tube 2, the core material 25 is forwardly passed through the through-hole 24. In the front vicinity of the extrusion ports 17 and 18 and front end opening 26, the core material 25 has externally fitted thereon the inner layer tube 2a of the tube 2, with the inner peripheral surface of the inner layer tube 2a being closely adhered thereto. Thereby, an intermediate product 47, which is a combination of the tube 2 and the core material 25, is molded. This intermediate product 47 is passed through another die 30, whereby molding is effected such that any cross-section of the tube 2 is perfectly circular and the outer diameter is constant. Further, thereafter, the intermediate product 47 is cold-cured by the cold curing device 13.

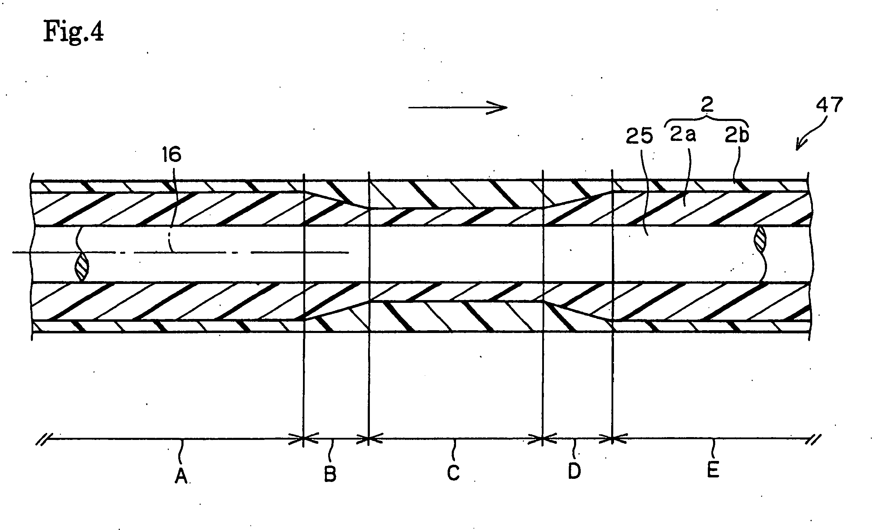

In Figs. 1 - 4, during molding of the intermediate product 47 by the extrusion molding apparatus 1, for example, as shown in Figs. 1 - 3, the valve body 39 of the first flow adjusting valve 34 is put in "full open position" and the valve body 39 of the second flow adjusting valve 35 is put in "half open position." Thereupon, the flow of the first resin 3 passed from the first extruder 6 through the first inflow passage 21 to the inner layer tube molding passage 9 is the full flow (QT) of the first resin 3 extruded from the first extruder 6, and increases more. On the other hand, the flow of the second resin 4 passed from the second extruder 7 through the second inflow passage 22 to the outer layer tube molding passage 10 is the remainder flow (Q2) of the second resin 4 extruded from the second extruder 7, and decreases more. Consequently, the tube 2 molded in said state, as shown by A and E in Fig. 4, has its inner layer tube 2a thick-walled and its outer layer tube 2b thin-walled.

Reversely to the above, the valve body 39 of said first flow adjusting valve 34 is put in "half open position." Further, the valve body 39 of said second flow adjusting valve 35 is put in "full open position." Thereupon, by the action reverse to the above, the tube 2, as shown by C in Fig. 4, has its inner layer tube 2a thin-walled and its outer layer tube 2b thick-walled.

As described above, in the case of putting the valve body 39 in "half open position," each of the flows of the resins 3 and 4 passed from the extruders 6 and 7 to the tube molding passages 9 and 10, is the reminder flow (Q2 = QT - Q1). However, since the partial flow (Q1) is discharged through said communication passage 43, the variation of the full flow (QT) of each of the resins 3 and 4 extruded from the extruders 6 and 7 is suppressed, and it is substantially constant. Here, when said valve body 39 is to be switched to either "full open position" or "half open position," more or less time is required for the turn R of the valve body 39. For this reason, as shown by B and D in Fig. 4, there are produced transition sections where the respective wall thicknesses of the inner and outer layer tubes 2a and 2b of the tube 2 longitudinally change.

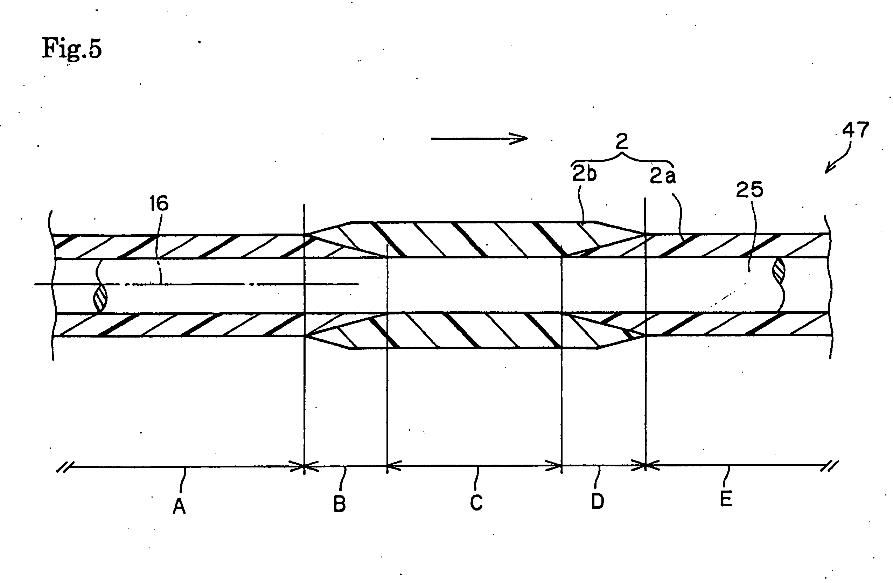

Referring to Figs. 1 - 3 and 5, during the molding of another intermediate product 47 by said extrusion molding apparatus 1, the valve body 39 of said first flow adjusting valve 34 is put in "full close position," with said another die removed from said die 11. Further, the degrees of opening of said inflow passages 21 and 22 are adjusted to 0. Thereupon, as shown by A and E in Fig. 5, the tube 2 is composed of the inner layer tube 2a alone. On the other hand, the valve body 39 of said first flow adjusting valve 34 is put in "full close position, " while the valve body 39 of said second flow adjusting valve 35 is put in "full open position, and the degree of openings of said inflow passages 21 and 22 are adjusted. Thereupon, as shown by C in Fig. 5, the tube 2 is composed of the outer layer tube 2b alone.

In the above case, another die 30 does not exist. For this reason, the front vicinity of the extrusion port 18 constituting the front end of said outer layer tube molding passage 10 is opened radially outward of said axis 16. Consequently, the outer diameter of the outer layer tube 2b can be made greater than that of the inner layer tube 2a. In other words, the outer diameter of the tube 2 can be adjusted to desired dimension at any longitudinal section. Further, the portions B and D in Fig. 5 are the same as the portions B and D of said Fig. 4.

Said intermediate product 47 is used as a material, for example, of catheters. That is, said intermediate product 47 is cut at predetermined longitudinal positions and into predetermined lengths by an unillustrated cutter. Thereafter, said core material 25 is longitudinally stretched by a stretching means, thereby being reduced in radial dimension. Then, this core material 25 is extracted from said tube 2 so as to separate said core material 25 fr0m the inner peripheral surface of the inner layer tube 2a of said tube 2, whereupon said catheter is molded.

Here, the first resin 3 of which the inner layer tube 2a of said tube 2 is molded differ in hardness from the second resin 4 of which the outer layer tube 2b is molded. For this reason, as shown in Figs. 4 and 5, the inner and outer layer tubes 2a and 2b in the tube 2 have their respective wall thicknesses and radial dimensions radially adjusted. Thereupon, the hardness and shape at any section of said tube 2 along the longitudinal direction can be continuously gradually changed, a fact which is convenient for molding catheters.

According to the above arrangement, the extrusion molding apparatus 1 comprises extruders 6 and 7 for thermally melting resins 3 and 4 to enable extrusion thereof, and a die 11 having tube molding passages 9 and 10 for forwardly passing therethrough the resins 3 and 4 extruded from the extruders 6 and 7 to make it possible to mold a tube 2, wherein flow adjusting valves 34 and 35 are installed which make it possible to adjust the flow per unit time of each of the resins 3 and 4 passing from said extruders 6 and 7 to the tube molding passages 9 and 10.

For this reason, in the case of molding the tube 2 by passing the resins 3 and 4, which are extruded from the extruders 6 and 7 by the driving of the extruders 6 and 7, through said tube molding passages 9 and 10, the flows of said resins 3 and 4 are adjusted by the actions attending the operation of said flow adjusting valves 34 and 35. Thereupon, the wall thickness and radial dimension of said tube 2 can be adjusted to desired values, so that a desired tube 2 is obtained.

Here, the volumes of the spaces in the "passages" for the flowing of the resins 3 and 4 extending from said flow adjusting valves 34 and 35 to the tube molding passages 9 and 10 is smaller than those extending from the extruders 6 and 7 to the tube molding passages 9 and 10. For this reason, the volumes of the resins 3 and 4 filling said "passages" are also small. Consequently, by an amount corresponding thereto, the volumetric variations of said resins 3 and 4 due to external force are suppressed such that they are small.

And, suppose that said flow adjusting valves 34 and 35 are actuated so as to change the flows of the resins 3 and 4 passing from said flow adjusting valves 34 and 35 to said tube molding passages 9 and 10. In this case, as described above, the volumes of the resins 3 and 4 in the "passages" are small and volumetric variations due to external force are suppressed such that they are small. For this reason, the changes of the flows of the resins 3 and 4 passing through said tube molding passages 9 and 10 follow the actuation of said flow adjusting valves 34 and 35 with satisfactory responsiveness. Consequently, the dimensional accuracy of the tube 2 being molded by the extrusion molding apparatus 1 can be made more accurate.

Further, as described above, said die 11 is formed with inflow passages 21 and 22 enabling the resins 3 and 4 extruded from the extruders 6 and 7 to flow into the rears of said tube molding passages 9 and 10, and the degrees of opening of said inflow passages 21 and 22 are made adjustable by said flow adjusting valves 34 and 35.

Here, the die 11 is formed with the tube molding passages 9 and 10 as described above, and the degrees of opening of the inflow passages 21 and 22 formed in said die 11 are made adjustable by said flow adjusting valves 34 and 35. For this reason, the flow adjusting valves 34 and 35 tend to be close to said tube molding passages 9 and 10. Consequently, the volumes of said "passages" become further small, and so do the volumes of the resins 3 and 4 filled in the "passages."

As a result, the changes of the flows of the resins 3 and 4 passing through said tube molding passages 9 and 10 follow the actuation of said flow adjusting valves 34 and 35 with satisfactory responsiveness. Consequently, the dimensional accuracy of the tube 2 being molded by the extrusion molding apparatus 1 can be made further accurate.

Further, said flow adjusting valve 34 and 35 are used for adjusting the degrees of opening of said inflow passages 21 and 22. Further, the inflow passages 21 and 22 are formed in the die 11. For this reason, said flow adjusting valves 34 and 35 can be simplified in arrangement by utilizing part of said die 11. In other words, said extrusion molding apparatus 1, though capable of accurately molding the tube 2, can be simplified in arrangement.

Further, as described above, the communication passage 43 for communicating the intermediate portions of said inflow passages 21 and 22 to outside said die 11 may be made openable/closable by said flow adjusting valves 34 and 35.

For this reason, when the resins 3 and 4 are passed from said extruders 6 and 7 through said flow adjusting valves 34 and 35 and said inflow passages 21 and 22 to said tube molding passages 9 and 10, the partial flows (Q1) of the full flows (QT) extruded from the extruders 6 and 7 are discharged in predetermined amounts to outside the die 11 through said communication passages 43 by said flow adjusting valves 34 and 35. Thereupon, this enables the adjustment of flows (remainder flows (Q2)) of the resins 3 and 4 passed to the tube molding passages 9 and 10.

That is, even in the case where the flows of the resins 3 and 4 passed to said tube molding passages 9 and 10are made adjustable, the full flows (QT) extruded from the extruders 6 and 7 can be made substantially constant. Here, whereas in the case of molding said tube 2 to desired dimensions, trying to change the extrusion flows from the extruders 6 and 7 would tend to make control troublesome, such control is unnecessary. Consequently, the molding of said accurate tube 2 described above is facilitated.

Further, as described above, there are installed opening-degree adjusting valves 44 which enable the adjustment of the degree of opening of the communications passages 43.

For this reason, the partial flows (Q1) of the resins 3 and 4 discharged to outside the die 11 through said communication passages 43 can adjusted to desired values by the adjustment of the degree of opening of the communication passages 43 due to said opening-degree adjusting valves 44. And, since such adjusting operation can be easily performed, the molding of the tube 2 of desired dimensions can be further facilitated.

Further, as described above, there are installed flow adjusting valves 34 and 35 which enable the adjustment of the respective flows per unit time of the resins 3 and 4 extruded from the extruders 6 and 7 and passed to said inner and outer layer tube molding passages 9 and 10.

For this reason, in the case of molding a multi-layer tube 2 by the driving of said extruders 6 and 7 to cause resins 3 and 4 extruded from the extruders 6 and 7 to pass through said tube molding passages 9 and 10, the flows of said resins 3 and 4 can be adjusted by the actions attending the operation on said flow adjusting valves 34 and 35. Consequently, the wall thicknesses and radial dimensions of said inner and outer layer tubes 2a and 2b can be adjusted to respective desired values, providing a desired multi-layer tube 2.

Here, the volumes of the spaces in the "passages" for the flowing of the resins 3 and 4 extending from said flow adjusting valves 34 and 35 to the tube molding passages 9 and 10 are smaller than those extending from the extruders 6 and 7 to the tube molding passages 9 and 10. For this reason, the volumes of the resins 3 and 4 filled in said "passages" become also small. Consequently, by the amount corresponding thereto the volumetric variations of said resins 3 and 4 due to external force are suppressed such that they are small.

And, suppose that said flow adjusting valves 34 and 35 are actuated so as to change the flows of the resins 3 and 4 passing from said flow adjusting valves 34 and 35 to said tube molding passages 9 and 10. In this case, as described above, the volumes of the resins 3 and 4 in the "passages" are small and volumetric variations due to external force are suppressed such that they are small. For this reason, the changes of the flows of the resins 3 and 4 passing through said tube molding passages 9 and 10 follow the actuation of said flow adjusting valves 34 and 35 with satisfactory responsiveness. Consequently, the dimensional accuracies of the inner and outer layer tubes 2a and 2b of the multi-layer tube 2 being molded by the extrusion molding apparatus 1 can be respectively made further high.

Further, as described above, the inner and outer extrusion ports 17 and 18 constituting the respective front ends of the inner and outer layer tube molding passages 9 and 10 are disposed close to each other radially of said axis 16, and are opened forwardly from the front end surface 19 of the die 11 separately from each other.

For this reason, the resins 3 and 4 extruded from the extruders 6 and 7 by the driving of the extruders 6 and 7 are passed through the tube molding passages 9 and 10 of the die 11, thereby molding the inner and outer layer tubes 2a and 2b. Further, when the inner and outer layer tubes 2a and 2b, which are extruded from said inner and outer extrusion ports 17 and 18, forwardly of the die 11, the outer layer tube 2b is externally fitted integrally on the inner layer tube 2a, so that a multi-layer tube 2 is molded.

In the above case, the inner and outer extrusion ports 17 and 18 are disposed radially close to each other. For this reason, when said resins 3 and 4 are passed through the tube molding passages 9 and 10 of said die 11 and extruded forwardly from the inner and outer extrusion ports 17 and 18, said inner and outer layer tubes 2a and 2b, said inner and outer layer tubes 2a and 2b immediately after they are forwardly extruded from said inner and outer extrusion ports 17 and 18 fit together without requiring relatively large radial deformation, and smoothly integrated.

Furthermore, as described above, the inner and outer extrusion ports 17 and 18 are partly or wholly opened forwardly from the front end surface 19 of said die 11 separately from each other. For this reason, when said inner and outer layer tubes 2a and 2b fit together, these inner and outer layer tubes 2a and 2b are suppressed from pressing each other.

Consequently, said inner and outer layer tubes 2a and 2b are prevented from being unintentionally deformed by mutual pressing. For this reason, the respective wall thicknesses of the inner and outer layers of the multi-layer tube 2 molded by said extrusion molding apparatus 1 can be respectively made more accurate.

Further, said inner and outer extrusion ports 17 and 18 extend along said axis 16 and substantially in parallel with each other.

For this reason, when said inner and outer layer tubes 2a and 2b immediately after they are forwardly extruded from said inner and outer extrusion ports 17 and 18 fit together, these inner and outer layer tubes 2a and 2b are more reliably suppressed from pressing each other. Consequently, said inner and outer layer tubes are prevented from being unintentionally deformed by mutual pressing. As a result, the respective wall thicknesses of said inner and outer layer tubes 2a and 2b can be made further accurate.

Further, as described above, said die 11 is formed with a through-hole 24 longitudinally extending through said die 11 and passing inwardly of said inner layer tube molding passage 9, said tube 2 being externally fitted on a core material 25 forwardly passing through said through-hole 24, and said inner extrusion port 17 of said inner layer tube molding passage 9 is disposed close to the front end opening 26 constituting the front end of said through-hole 24 radially of said axis 16.

For this reason, extruded from said die 11 by the driving of said extruders 6 and 7, a multi-layer tube 2 is molded, this tube 2 being externally fitted on said core material 25, thus molding an intermediate product 47 using the tube 2 and the core material 25.

Here, as described above, the inner extrusion port 17 is disposed radially close to said front end opening 26. Furthermore, as described above, the inner and outer extrusion ports 17 and 18 are disposed radially close to each other. For this reason, when said tube 2 is extruded from said inner extrusion port 17 forwardly of the die 11, said inner and outer layer tubes 2a and 2b immediately after they are extruded from said inner extrusion port 17 are, without requiring large radial deformation, externally fitted on the core material 25 immediately after slipping out of the front end opening 26 in said through-hole 24.

Consequently, the multi-layer tube 2 in said intermediate product 47 molded by said extrusion molding apparatus 1 can have the respective wall thicknesses of its inner and outer layer tubes 2a and 2b made more accurate.

In addition, what has so far been described is by way of illustrated examples. Said tube 2 and tube molding passages 9 and 10 may be single-layered or three- or more-layered. Further, any one of the inner and outer layer tubes 2a and 2b of the tube 2 may have its hardness increased. Further, a gear pump may be interposed between said extruders 6 and 7 and die 11. Further, said flow adjusting valves 34 and 35 may be interposed between said extruders 6 and 7 and die 11.

Further, the invention may be achieved by suitably combining individual component members described above.

## Claims

1. An extrusion molding apparatus for a resin tube, comprising a plurality of extruders (6, 7) for thermally melting and extruding resins (3, 4) of different kinds, and a die (11) provided with an inner layer tube molding passage (9) for forwardly passing therethrough the resin (3) extruded from one extruder (6) of these extruders (6, 7) to enable the molding of an inner layer tube (2a), and an outer layer tube molding passage (10) for forwardly passing therethrough the resin (4) extruded from the other extruder (7) to enable the molding of an outer layer tube (2b) which is to be externally fitted integrally on said inner layer tube (2a), said die (11) enabling the molding of a multi-layer tube (2) by these inner and outer layer tubes (2a, 2b), said die (11) being formed with inflow passages (21, 22) enabling the resins (3, 4) extruded from said extruders (6, 7) to flow into the respective rears of said tube molding passages (9, 10), wherein flow adjusting valves (34, 35) are installed which enable the adjustment of the respective flows per unit time of the resins (3, 4) extruded from said extruders (6, 7) and passed said inner and outer layer tube molding passages (9, 10),
said flow adjusting valves (34, 35) make openable/closable the communication passage (43) for communicating the intermediate portion of said inflow passages (21, 22) to outside said die (11 ), said extrusion molding apparatus for a resin tube being **characterized in that**
opening-degree adjusting valves (44) are installed which enable the adjustment of the respective degrees of opening of said communication passages (43).

2. An extrusion molding apparatus for a resin tube as set forth in claim 1, **characterized in that** inner and outer extrusion ports (17, 18) constituting the respective front ends of said inner and outer layer tube molding passages (9, 10) are disposed radially close to each other and are opened forwardly of the front end surface (19) of the die (11) and separately from each other.

3. An extrusion molding apparatus for a resin tube as set forth in claim 2, with said die (11) formed with a through-hole (24) longitudinally extending through said die (11) and extending inwardly of said inner layer tube molding passage (9), said tube (2) being externally fitted on the core material (25) forwardly passing through said through-hole (24),
**characterized in that** said inner extrusion port (17) of said inner layer tube molding passage (9) is disposed close to the front end opening (26) radially constituting the front end of said through-hole (24).

## Patentansprüche

1. Extrusionsformvorrichtung für eine Harzröhre mit einer Vielzahl von Extrudern (6, 7) zum thermischen Schmelzen und Extrudieren von Harzen (3, 4) unterschiedlicher Arten und einem Düsenkopf (11), der versehen ist mit einem Innenschichtröhrenformungsdurchgang (9) zum Durchführen des von einem Extruder (6) dieser Extruder (6, 7) extrudierten Harzes (3) dorthindurch nach vorn, um das Formen einer Innenschichtröhre (2a) zu ermöglichen, und mit einem Außenschichtröhrenformungsdurchgang (10) zum Durchführen des von dem anderen Extruder (7) extrudierten Harzes (4) dorthindurch nach vorn, um das Formen einer Außenschichtröhre (2b) zu ermöglichen, die von außen auf die Innenschichtröhre (2a) aufzupressen ist, wobei der Düsenkopf (11) das Formen einer mehrschichtigen Röhre (2) durch diese Innen- und Außenschichtröhren (2a, 2b) ermöglicht, wobei der Düsenkopf (11) mit Einflussdurchgängen (21, 22) ausgebildet ist, die ermöglichen, dass die von den Extrudern (7, 8) extrudierten Harze (3, 4) in die jeweiligen Rückseiten der Röhrenformungsdurchgänge (9, 10) fließen, wobei Flusseinstellventile (34, 35) installiert sind, die die Einstellung der jeweiligen Flüsse pro Einheitszeit der von den Extrudern (6, 7) extrudierten und durch die Innen- und Außenschichtröhrenformungsdurchgänge (9, 10) geführten Harze (3, 4) ermöglichen, wobei die Flusseinstellventile (34, 35) veranlassen, dass der Kommunikationsdurchgang (43) zum Kommunizieren des mittleren Teilbereichs der Einflussdurchgänge (21, 22) zur Außenseite des Düsenkopfs geöffnet/geschlossen werden kann, wobei die Extrusionsformvorrichtung für eine Harzröhre **dadurch gekennzeichnet ist, dass**
Offnungsausmaß-Einstellventile (44) installiert sind, die die Einstellung der jeweiligen Öffnungsausmaße der Kommunikationsdurchgänge (43) ermöglichen.

2. Extrusionsformvorrichtung für eine Harzröhre nach Anspruch 1, **dadurch gekennzeichnet, dass** Innen- und Außenextrusionstore (17, 18), die die jeweiligen vorderen Enden der Innen- und Außenschichtröhrenformungsdurchgänge (9, 10) bilden, radial nahe zueinander angeordnet sind und von der Oberfläche (19) des vorderen Endes des Düsenkopfs (11) nach vorn und getrennt voneinander geöffnet sind.

3. Extrusionsformvorrichtung für eine Harzröhre nach Anspruch 2, wobei der Düsenkopf (11) mit einem Durchgangsloch (24) ausgebildet ist, das sich längs durch den Düsenkopf (11) erstreckt und sich von dem Innenschichtröhrenformungsdurchgang (9) nach innen erstreckt, wobei die Röhre (2) von außen auf dem Kernmaterial (25) aufgepresst ist, das durch das Durchgangsloch (24) nach vorn durchläuft,
**dadurch gekennzeichnet, dass** das Innenextrusionstor (17) des Innenschichtröhrenformungsdurchgangs (9) nahe der Öffnung (26) des vorderen Endes angeordnet ist, die das vordere Ende des Durchgangslochs (24) bildet.

## Revendications

1. Appareil de moulage par extrusion d'un tube en résine, comprenant une pluralité d'extrudeuses (6, 7) pour faire fondre thermiquement et extruder des résines (3, 4) de différentes sortes, et une filière (11) munie d'un passage de moulage de tube à couche interne (9) pour faire passer vers l'avant à travers celui-ci la résine (3) extrudée d'une extrudeuse (6) de ces extrudeuses (6, 7) afin de permettre le moulage d'un tube à couche interne (2a), et un passage de moulage de tube à couche externe (10) pour faire passer vers l'avant à travers celui-ci la résine (4) extrudée de l'autre extrudeuse (7) afin de permettre le moulage d'un tube à couche externe (2b) destiné à être emboîté de manière solidaire depuis l'extérieur sur ledit tube à couche interne (2a), ladite filière (11) permettant le moulage d'un tube multicouche (2) par ces tubes à couches interne et externe (2a, 2b), ladite filière (11) étant formée avec des passages d'admission (21, 22) permettant aux résines (3, 4) extrudées desdites extrudeuses (6, 7) de s'écouler dans les parties arrières respectives desdits passages de moulage de tube (9, 10), dans lesquelles sont installées des soupapes régulatrices de débit (34, 35) qui permettent d'ajuster les débits respectifs par unité de temps des résines (3, 4) extrudées desdites extrudeuses (6, 7) et passées à travers lesdits passages de moulage de tube à couche interne et externe (9, 10),
lesdites soupapes régulatrices de débit (34, 35) permettent ouverture/fermeture pour le passage de communication (43) faisant communiquer la portion intermédiaire desdits passages d'admission (21, 22) avec l'extérieur de ladite filière (11), ledit appareil de moulage par extrusion d'un tube en résine étant **caractérisé en ce que**
des soupapes de réglage de degré d'ouverture (44) sont installées, qui permettent de régler les degrés d'ouverture respectifs desdits passages de communication (43).

2. Appareil de moulage par extrusion d'un tube en résine selon la revendication 1, **caractérisé en ce que** des ports d'extrusion interne et externe (17, 18) constituant les extrémités avant respectives desdits passages de moulage de tube à couche interne et externe (9, 10) sont disposés radialement à proximité l'un de l'autre et sont ouverts vers l'avant par rapport à la surface d'extrémité avant (19) de la filière (11) et séparément l'un de l'autre.

3. Appareil de moulage par extrusion d'un tube en résine selon la revendication 2, ladite filière (11) étant formée avec un orifice de passage (24) traversant longitudinalement ladite filière (11) et s'étendant vers l'intérieur dudit passage de moulage de tube à couche interne (9), ledit tube (2) étant emboîté depuis l'extérieur sur le matériau de noyau (25) passant vers l'avant à travers ledit orifice de passage (24),
**caractérisé en ce que** ledit port d'extrusion interne (17) dudit passage de moulage de tube à couche interne (9) est disposé à proximité de l'ouverture d'extrémité avant (26) constituant radialement l'extrémité avant dudit orifice de passage (24).
